Europäisches Patentamt

European Patent Office

Office européen des brevets

(11) Veröffentlichungsnummer: **0 588 145 A2**

# EUROPÄISCHE PATENTANMELDUNG

(21) Anmeldenummer: **93113881.2**

(22) Anmeldetag: **31.08.93**

(51) Int. Cl.5: **A61L 2/18**, C12N 1/00

(30) Priorität: **09.09.92 CH 2837/92**

(43) Veröffentlichungstag der Anmeldung:
**23.03.94 Patentblatt 94/12**

(84) Benannte Vertragsstaaten:
**AT BE DE ES FR GB IE IT LU NL**

(71) Anmelder: **SYLVAN PILZ AG**
**Tannenbergstrasse**
**CH-8625 Gossau-Zürich(CH)**

(72) Erfinder: **Stadelmann, René J., Dr.**
**Langenmattstrasse 34**
**CH-8617 Mönchaltorf(CH)**

(74) Vertreter: **Fillinger, Peter, Dr.**
**Rütistrasse 1a,**
**Postfach 358**
**CH-5401 Baden (CH)**

(54) **Verfahren zur Aufbereitung eines festen Nährsubstrates für Mikroorganismenkulturen in Reinkultur.**

(57) Die neue Erfindung schlägt eine wesentlich schonendere Aufbereitung eines festen Nährsubstrates für die Kultivierung von Mikroorganismenkulturen in Reinkultur vor. Die Kornmasse wird vorgängig der Aufquellung in einem Prozessraum (3), in mühlentechnischen Sortier- und Reinigungseinrichtungen (56) gereinigt. Die Aufquellung erfolgt je nach Kornsorte im Überschusswasser bei 50 bis 99 ° C, welche zusammen mit einer chemischen Oberflächensterilisierung erfolgt. Die chemische Behandlung er-gibt zusammen mit der Wärme ohne die herkömmlich angewandten Dampfsterilisationstemperaturen, ein steriles Nährsubstrat. Den Körnern können mit einem zwei- oder sogar mehrfachen Beschichtungsverfahren Haft- und Nährsubstanzen und deshalb eine viel dickere Puderschicht aufgetragen werden. Das Brutmaterial ist in gebrauchsfertigem Zustand sehr gut rieselfähig, das Myzel wächst darauf optimal und das Verfahren ist zudem wesentlich wirtschaftlicher betreibbar.

FIG. 1

EP 0 588 145 A2

Die Erfindung betrifft ein Verfahren gemäss dem Oberbegriff des Anspruchs 1.

In der vorliegenden Beschreibung wird der Begriff Sterilisieren für chemische und physikalische Massnahmen verwandt, die der Abtötung sämtlicher Keime innerhalb eines Raumes dienen. Zum Sterilisieren im Sinne dieser Anmeldung können somit auch chemische Desinfektionsmittel verwendet werden, sofern damit die Abtötung aller Fremdkeime und Mikroorganismen erreicht wird.

Nährsubstrat bildet im Brutmaterial die wesentliche Ernährungsgrundlage für dessen Mikroorganismus. An solches Brutmaterial werden verschiedene Grundforderungen gestellt. Für Brutmaterial einer Champignonzucht z.B. muss das Nährsubstrat keimfrei, d.h. mit keinen Mikroben, Sporen oder dgl. Mikroorganismen infiziert sein. Nur so wird nach dem Impfen des Nährsubstrats mit dem gewünschten Myzel (genannt Inoculierung) einwandfreies Brutmaterial (Reinkultur) für die z.B. zu züchtenden Speisepilze erhalten. Der angestrebte Wassergehalt im Nährsubstrat liegt im Falle der Produktion von Brutmaterial für Kultur-Champignons je nach Sorte bei 40 bis 50 %. Zudem sollten die Nährstoffe des Nährsubstrates bis zum Beginn des Myzelwachstums ungeschmälert erhalten bleiben. Eine gleichmässige Aussaat von Brutmaterial erfordert eine gleichmässige, streufähige Beschaffenheit. Als Nährsubstrate werden vorwiegend Früchte bzw. Samen von grünen Pflanzen, bevorzugt Getreidekörner, verwendet. Gleichzeitig mit der Aufbereitung des Nährsubstrates muss deshalb die Keimfähigkeit solcher Samen ausgeschaltet werden, damit nicht der Keimling z.B. des Getreidekornes in Konkurrenz zu dem Pilzmyzel für den Abbau der Nährstoffe des Kornes treten. Sehr stark verbreitet ist die Verwendung von Roggen- oder Hirsekörnern als Basissubstrate.

In der US-PS Nr. 2 932 862 ist das zur Zeit fortschrittlichste Verfahren zur Herstellung von Brutmaterial für die Produktion von Speisepilzen beschrieben. Gemäss dieser Druckschrift wird das Nährstoffgemisch (Ausgangskornmaterial) bei einer Temperatur von mindestens 122° C während 35 bis 120 Minuten sterilisiert. Dies geschieht unter Anwendung von Wasser und Heissluft bzw. Heissdampf unter Druck in einem V-förmigen Trommelmischer. Anschliessend wird das sterile und aufgequollene Korngut abgekühlt und bildet das Nährsubstrat; dieses wird mit dem gewünschten Organismus geimpft, gemischt und in einen Inkubationsbehälter abgefüllt. Nachteile dieser Methode liegen darin, dass z.B. wegen der hohen Sterilisationstemperatur Körner platzen oder reissen können, die dann zu Ausgangspunkten für kleine Klumpen führen. Auch können bei diesen hohen Sterilisationstemperaturen und langen Sterilisationszeiten Nährstoffe im Substrat zerstört oder umgewandelt werden, was sich hierauf auf das Wachstum des Myzels nachteilig auswirken kann.

Ein weiterer Nachteil dieser bekannten Sterilisation bei mindestens 122° C liegt darin, dass die Masse an Ausgangskornmaterial nach der Aufbereitung wegen dessen schlechter Wärmeleitfähigkeit viel länger im Mischer gekühlt werden muss bis die Impftemperatur < 30° C erreicht wird.

Der Erfindung wurde nun die Aufgabe gestellt, das bekannte Verfahren zu verbessern, insbesondere eine ökonomischere Aufbereitung von grösseren Mengen von z.B. 2 bis 5 t zu ermöglichen, ohne dass qualitative Nachteile für das Nährsubstrat entstehen.

Die erfindungsgemässe Lösung ist gekennzeichnet, durch die Merkmale des Anspruchs 1.

Die Erfinder erkannten, dass bei allen bisherigen Ansätzen für die Herstellung eines Nährsubstrates für Brutmaterial sehr wesentliche Tatsachen übersehen wurden. Eine thermische Sterilisation des Ausgangskornmaterials wie bei Lebensmitteln üblich wurde für die Herstellung von Brutmaterial als unzweckmässig erkannt. Das thermische Sterilisieren von Lebensmitteln wird für eine möglichst lange Konservierung durchgeführt, damit alle möglichen Vorgänge, insbesondere auch enzymatische Abläufe oder der langsame Abbau von Fetten und Ölen, bei Raumtemperatur, möglichst lange verzögert werden. Bei dem Nährsubstrat für Mikroorganismenkulturen müssen aber keine solchen Veränderungen im Inneren des Kornes thermisch erzwungen und damit eine unkontrollierte Zersetzung in Kauf genommen werden. Im Gegenteil, bei Brutmaterial wird eine rasche Verwertung gewünscht. Nur dazu ist das Korn vorzubereiten. Eine chemische Oberflächensterilisation der in Wasserüberschuss gequollenen Körner genügt, wenn die Körner zuvor gereinigt wurden und ihre Fruchtschale nicht zerstört wurde. Es genügt also eine Sterilisation der Kornaussenpartien, die den gleichen Nutzeffekt hat wie eine durchgehende Erhitzung der Körner bei einer Temperatur von mindestens 122° C. Das neue Verfahren erlaubt die Aufbereitung von sehr grossen Mengen, sei es im Batch- oder im kontinuierlichen Verfahren und insbesondere eine ökonomischere Betriebsweise durch drastische Verkürzung der Verfahrenszeiten.

Als weiterer Vorteil ergab sich ein homogeneres Nährsubstrat und daher ein riesel- oder streufähigeres Brutmaterial. Durch die relativ geringe Hitzebelastung der intakten Getreidekörner werden nahezu keine geplatzten Körner festgestellt. Ferner kann die Bräunung bei Getreidekörnern, die bei Temperaturen von 122° C und mehr eintritt, und zusätzlich eine entsprechende Verminderung der Nährwerte des Kornes verursacht, vermieden werden. Durch das sehr schonende Aufquellen und chemische Sterilisieren bei Temperaturen unter 100° C bleibt der Kornaufbau unbeschädigt. Das Korngut kann zudem mit einer gleichmässigen Schicht einer vorsterilisierten Haftsubstanz überzogen werden. Diese kann z.B. aus Mischfettsäuren bestehen, wie sie als Nebenprodukte bei der Speiseölherstellung anfallen. Diese wiederum kann zusätzlich mit Schutz- und Wachstumsförderstoffen für das Myzel angereichert werden. Es hat sich ferner gezeigt, dass gegenüber den bekannten Verfahren die Körner mit einem Haftüberzug sich mit einer viel grösseren Menge und zudem gleichmässiger mit einem kalkhaltigen Puder überziehen lassen.

Das Wachstum von Pilzmyzel nimmt im Bereiche eines PH-Wertes von 6,5 bis 7 den besten Verlauf. Erfindungsgemäss ist es problemlos möglich, das Mengenverhältnis von Fettsäuremantel und Kalkpudermantel auf diese Werte opitmal einzustellen. Solche für das Myzelwachstum nährstoffreiche Beschichtungen bewirken, dass das Myzel nach der Impfung rascher in Richtung der Mantelschicht wächst und erst mit Verzögerung in das Korninnere eindringt. Dadurch wird eine gleichmässigere Umwachsung des einzelnen Kornes durch das Myzel und eine gleichmässigere Durchwachsung des Brutmaterials erreicht.

Die Erfindung wird in der Folge anhand einiger Ausführungsbeispiele mit weiteren Einzelheiten erläutert. Es zeigen:

Fig. 1    Den Aufbereitungsteil als Ausschnitt aus der Anlage gemäss Fig. 4 für das Nährsubstrat;

Fig. 2    die Wasseraufnahme von Roggen in Abhängigkeit von Temperatur und Zeit;

Fig. 3    zwei Beispiele für den Prozessverlauf einer vollständigen Nährsubstrataufbereitung und Inoculierung mit Roggen als Ausgangsmaterial;

Fig. 4    das Schema einer vollständigen Anlage für die Herstellung von Brutmaterial für die Pilzzucht;

Fig. 5    ein Schema für die Führung der verschiedenen Medien;

Fig. 6    Detail VI aus der Anlage gemäss Fig. 4 für die Inoculierung von kleinen und mittleren Nährsubstratmengen und/oder Herleitung von Impfmaterial und

Fig. 7    ein Blockdiagramm zum Verfahrensablauf bei einer Anlage nach Fig. 4.

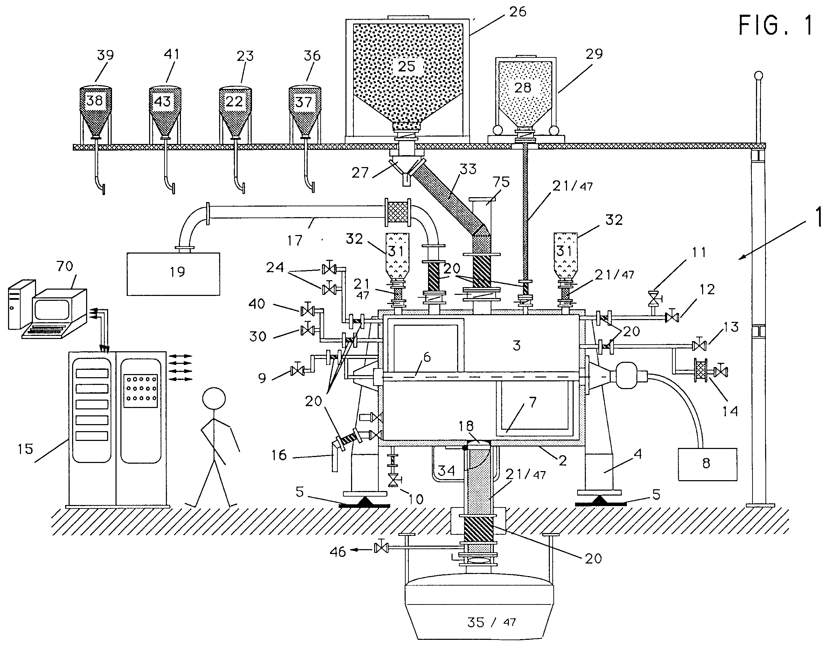

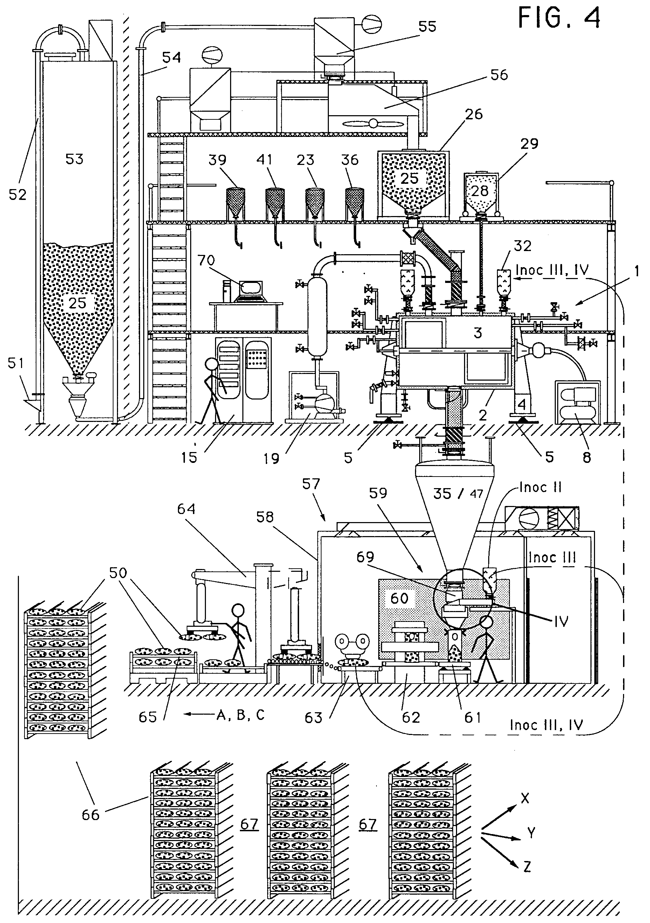

In der Folge wird nun auf die Fig. 1 und 4 Bezug genommen, deren zentraler Teil eine in Fig. 1 herausgestellte Aufquell- und Sterilisiereinheit 1 ist. Diese weist einen, nach aussen vollständig abschliessbaren Mantel 2 auf, der einen Prozessraum 3 umfasst und über Konsolen 4 auf Wägezellen 5 abgestützt ist. Im Inneren des Prozessraumes 3 befinden sich an einer horizontalen Hohlwelle 6 hohle Rührarme 7, welche über ein schematisch dargestelltes Hydraulikgetriebe 8 in Umlauf versetzbar sind. Der Mantel 2 ist als Hohlmantel ausgebildet und zusammen mit der Hohlwelle 6 und den hohlen Rührarmen 7 an einen Temperatursteuerungskreislauf angeschlossen, so dass je nach Bedarf über steuerbare Absperrelemente 9, Kühl- oder Heizmedien in den Hohlmantel 2 bzw. in die Hohlwelle 6 und die Rührarme 7 eingelassen und über einen weiteren ebenfalls steuerbaren Auslass 10 wieder abgeführt werden können. Zur periodischen Reinigung des Prozessraumes 3, insbesondere zur Entfernung von Ablagerungen lässt sich aus einem Reinigungskreislauf wahlweise Spülwasser über einen Spülwasserschieber 11 und Heisswasser über einen Heisswasserschieber 12 einfüllen, wobei alle Steuereingriffe, auch die in der Folge beschriebnen, über eine zentrale Steuerung 15 kontrolliert und befohlen werden können. Im Prozessraum 3 befindliches flüssiges Medium lässt sich über einen Bodenauslass 16 und gasförmiges Medium über eine Aspiration 17 und eine nachgeschaltete Hochvakuumanlage 19 ableiten.

Damit die in den Behandlungsraum 3 der Aufquell- und Sterilisiereinheit 1 eingegebenen Medien tatsächlich darin gewogen werden können, müssen alle festen Anschlüsse nach aussen über flexible Rohre respektive flexible Schläuche 20 mit dem Mantel 2 verbunden sein. Zumindest die grossquerschnittigen Produkt-Zu- und Abführungen weisen im Nahbereich des Mantels 2 eine Schleuse 21 inkl. Drehschieber auf. Diese sind mit einem Spülsystem mit Sprühdüsen 47 versehen (hier nicht dargestellt), so dass periodisch auch die entsprechenden sonst schlecht zugänglichen Räume rasch und automatisch keimfrei reinigbar sind.

Geeignetes Desinfektionsmittel 22 sowie Reinigungsmittel 37 können von Tauschcontainern 23 bzw. 36 über verschliessbare Hahnen 24 eines entsprechenden Kreislaufes in den Prozessraum 3 gespritzt werden. Nach dem Abführen des Endproduktes kann zur Vervollständigung der Sterilisation des Behandlungsraumes 3 und der übrigen Infrastruktur (Schieber, Schleusen und dgl.) Dampf über einen Dampfschieber 13 und Sterilluft über einen Sterilluftschieber 14 eingefüllt werden. Die Aufquell- und Sterilisiereinheit 1 kann auch aus zwei parallelen Einheiten in Einzel- oder Zwillingsbauweise konzipiert sein.

Als Ausgangskornmaterial 25 für das Nährsubstrat sind bei einer Anlage nach Fig. 1 gereinigte Roggenkörner vorgesehen, welche in einem Vorcontainer 26 chargenweise bereitstellbar sind. Der Gesamtbesatz bzw. derBruchkornanteil muss kleiner sein als 5 % bzw. 3 %. Die Körner fliessen unter der Schwerkraft über eine regelbare Dosierklappe 27, ein Kornzuführrohr 33 und einen flexiblen Verbindungsschlauch 20 in den Prozessraum 3. Die Dosiermenge wird über entsprechende Gewichtssignale von den Wägezellen 5 über die Steuerung 15 bestimmt. Der für das Beschichten (Coaten) der Körner benötigte Kalkpuder 28 ist in einem Puderbehälter 29 bereitgestellt und wird über eine ebenfalls sterilisierbare Verbindungsleitung als Schleuse 21 ausgeführt in den Prozessraum 3 geleitet. Dieser Vorgang lässt sich z.B. mit dem von der Vakuumanlage 19 im Prozessraum 3 erzeugten Unterdruck unterstützen. Zusätzliche Präparate können ebenfalls eingeführt werden. Beispielsweise können vor der Puderzugabe Mischfettsäuren 38 aus einem Behälter 39 mittels eines Hahns 40 eingeführt und damit zusätzlich die Nährwerte des Nährsubstrats für das Myzel verbessert und eine die einzelnen Körner überziehende Haftschicht für den Puder und ein optimaler pH-Wert erreicht werden. Die Zuführung lässt sich ebenfalls mit der Vakuumanlage 19 unterstützen. Ferner lassen sich weitere Prozesschemikalen 43, welche die chemische Sterilisation unterstützen, aus dem Container 41 mittels des Hahns 30 in den Prozessraum 3 eindosieren. Neben dem Ausgangskornmaterial 25, dem Aufquellwasser und dem Puder 28 ist die eigentliche Impfmasse respektiv das Inoculum 31, welches in Inocbomben 32 bereitgestellt ist, die Hauptkomponente, die mit dem Ausgangskornmaterial vermischt werden soll. Das Inoculum besteht aus Körnern oder andern rieselfähigen, festen Nährsubstraten, die bereits vollständig mit dem Myzel bewachsen sind. Das Beimischen des Inoculums 31 kann, wie in der Fig. 1 dargestellt ist, direkt aus einer Inocbombe 32 in den Prozessraum 3 erfolgen.

Da sowohl für das Aufquellen wie für das Sterilisieren in engen Grenzen festlegbare Parameter wie Zeit, Temperatur usw. eingehalten werden müssen, wird dieser Kernteil des Verfahrens vorzugsweise als sog. Batchbetrieb respektive Chargenbetrieb durchgeführt. Damit können insbesondere für jedes Partikel des Nährsubstrates gleiche Bedingungen eingehalten werden, so dass auch das Endprodukt einen hohen Grad an Gleichmässigkeit besonders auch für den Wassergehalt aufweist. Am Ende der ganzen Batchdauer kann das sterile Nährsubstrat ggf. das Brutmaterial über ein Austragsystem 34 über eine sterilisierbare Schleuse 21 und eine flexible Verbindungsleitung 20 in einen Zwischenspeicher 35 abgefüllt werden.

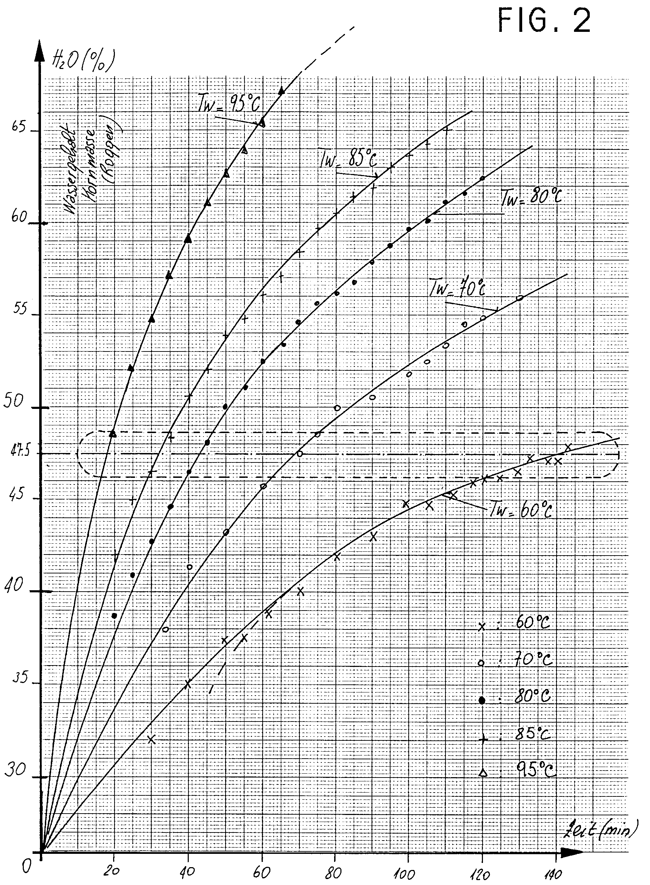

In Versuchsreihen wurde bei verschiedenen Wassertemperaturen die Abhängigkeit der Wasseraufnahme durch Roggen (Aufquellprozess von der Zeit ermittelt und aufgezeichnet (Fig. 2). Augenfällig dabei ist, dass eine relativ strenge Gesetzmässigkeit vorhanden ist. Der Endfeuchtegehalt des Nährsubstrats hängt von der zu impfenden Myzelsorte ab. Generell ist es das Ziel, dass das Nährsubstrat einen Wassergehalt von 40 bis 60 %, vorzugsweise 42 bis 52 % besonders vorzugsweise 48 oder 43 % bei der Impfstoffzugabe von Myzelkulturen zur Pilzbrut-Herstellung aufweist, da dann die Wachstumsbedingungen für die Myzelien der gängigsten Sorten optimal sind. Derselbe Roggen benötigte bei einer Wassertemperatur von 60° C 140 Minuten, bei einer Wassertemperatur von 95° C dagegen nur 20 Minuten, bis er einen Wassergehalt von 47,5 % erreichte. Der Fachmann weiss, dass das Getreide, eine Pflanzenfrucht, sogar innerhalb derselben botanischen Sorte aber einer anderen Ernte unterschiedlich lange Zeiten z.B. für die Aufnahme einer exakten Wassermenge beim Quellen benötigt. Dieser Tatsache muss bei der Steuerung des Verfahrens Rechnung getragen werden.

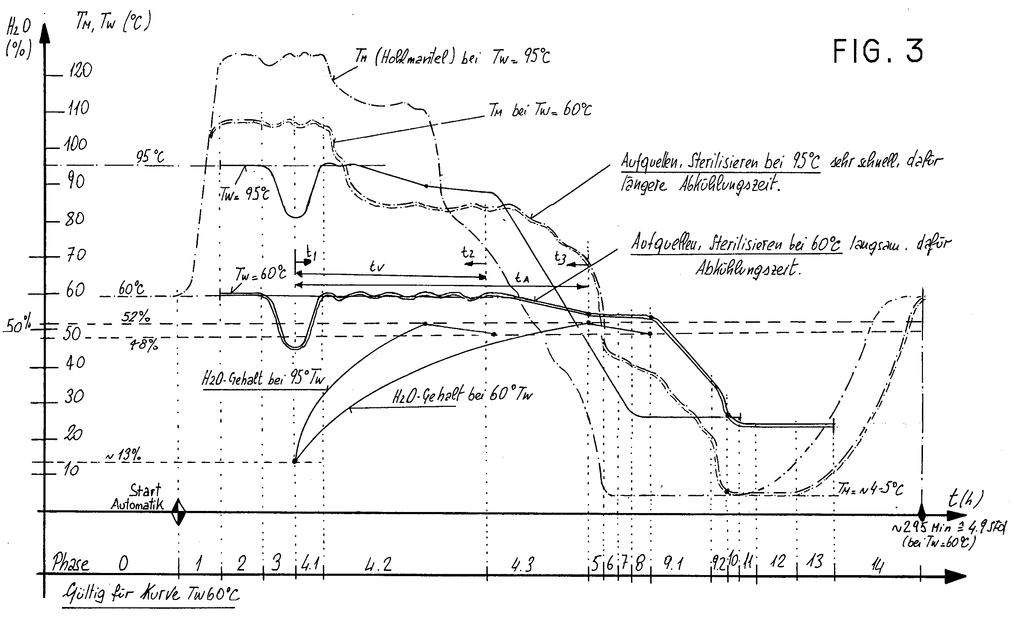

In Fig. 3 sind zwei Beispiele des Prozessverlaufs für eine ganze Batchdauer aufgrund von Versuchswerten dargestellt. Dabei sind zwei unterschiedliche Temperaturen (Aufquelltemperatur), nämlich 60° C und 95° C für das Wasser des Aufquellvorganges aufgezeichnet worden. Diese Temperaturen sind als "empfohlene Unter- und Oberwerte" zu verstehen. Die ideale Temperaturführung liegt in dieser Spanne und hängt vorwiegend von den Anfangskornwerten ab (Sorte, Ernte, Wasseraufnahme, etc.). Die Versuche haben bestätigt, dass, bedingt durch den schlechten K-Wert des Roggens bei der Verarbeitung von Mengen in industriellem Massstab (z.B. 2 bis 10 t), für die Abkühlung aus sterilitäts- und messtechnischen Gründen kein kaltes Spülwasser verwendet werden darf, besonders viel Zeit beansprucht. Folglich läuft mit steigenden Temperaturen das Aufquellen schneller und das Abkühlen langsamer ab. Die Fig. 3 zeigt auch, dass die Abkühlung der Körner unter Vakuum wegen der damit entnommenen Verdampfungswärme wesentlich schneller erfolgt als wenn nur der Hohlmantel 2, die Hohlwelle 6 und die Rührarme 7 gekühlt werden.

Eine andere Temperaturführung, insbesondere über 95° C, als die in Fig. 3 gezeigte ist in der Aufquell- und Sterilisiereinheit 1 möglich, doch dürfen dabei keine thermisch bedingten Schäden an den Körnern auftreten.

Fig. 4 zeigt eine vollständige Anlage für die Herstellung von in vorsterilisierten Brutsäcken 50 abgefülltem Brutmaterial. Ausgangskornmaterial 25 für das Nährsubstrat ist z.B. handelsüblicher, sauber gereinigter Roggen oder Hirse, welcher über einen Annahmetrichter bzw. Normanschluss 51 und über eine pneumatische Förderanlage 52 in einem Roggensilo 53 gelagert wird. Die für ein Batch benötigte Menge Roggen wird über einen weiteren pneumatischen Förderer 54 über einen Abscheider 55 einer trockenen Reinigungseinrichtung 56 zugeführt. Die trockene Reinigung soll noch vorhandenen Fremdbesatz, Schalen, Schmutz und Staub und auch Kornbruch auslesen; sie soll aber so schonend für das Korn durchgeführt werden, dass kein neuer Bruch und keine Beschädigung der Kornschalen entsteht, da die ganze Schale in mehrfacher Hinsicht für die Qualität des Brutmateriales wichtig ist. Es ist erwünscht, dass gleichzeitig mit der Reinigung die Keimzahl der Kornmasse reduziert wird. Je nach Grösse der Anlage ist es möglich, die gesamte Getreidereinigung in die Nährsubstrataufbereitung zu integrieren oder aber die entsprechenden Einrichtungen einer Getreidemühle zu verwenden, damit die genannten Grenzwerte für Besatz und Bruchkorn eingehalten sind. Die gesamte Getreideförderanlage ist dicht geschlossen, um Raumkontaminationen zu vermeiden. Gemäss Fig. 4 ist die Anlage als Kompaktanlage in einem vier Etagen aufweisenden Stahlgerüst aufgebaut, wobei die Getreidereinigung auf der dritten und vierten Etage montiert ist. ReinigungsmittelTauschcontainer 36, Puderbehälter 29, Zusatzmittelbehälter 39, Desinfektionsmittel-Tauschcontainer 23 sowie Prozesschemikalien-Container 41 sind auf der zweiten Etage aufstellbar.

Die Aufquell- und Sterilisiereinheit 1 ruht auf Wägezellen 5 direkt auf der speziell schwingungsfrei angelegten Betonplatte.

In einem Untergeschoss befindet sich eine Abfüllanlage 57, in der das behandelte und frisch inoculierte Brutmaterial in vorsterilisierte Brutsäcke 50 abgefüllt wird. Die gängige Praxis bei der Herstellung von Pilzbrutmaterial geht so vor sich, dass in ein Nährsubstrat z.B. in Form einer Masse von gereinigten, sterilen und gequollenen Roggenkörnern, eine kleine Menge mit Myzel bewachsenen Körner eingemischt wird. Nach dieser Impfung (Inoculierung) sind nach einer bestimmten Zeit, z.B. nach 8 bis 21 Tagen, in Brutsäcken 50 oder Plastikcontainern alle Körner gleichmässig überwachsen und bereit, für das Einbringen in den Kompostnährboden einer Pilzzuchtanstalt. Damit nun aber bei der Abfüllung nicht Fremdkeime wie Bakterien oder Pilzsporen in das Brutmaterial gelangen, wird der ganze Abfüllvorgang in einer Reinraumkabine 58 durchgeführt. Innerhalb der Reinraumkabine 58 kann im Nahbereich einer Sackfüllstation 59 eine innere sterile Zone 60 z.B. durch die Einbringung von steriler Luft der Klasse 100 (U.S. Fed. Std. 2096) geschaffen werden (Laminar-Flow). In dem Zwischenspeicher 35, der im wesentlichen die Form eines nach unten verjüngten Konus hat, ist am unteren Auslass ein von einer elektronischen Waage 61 steuerbares Dosiersystem 69 angeordnet, das bei schonender Behandlung, genaue Portionen z.B. von 2 bis 15 kg Brutmaterial in jeden einzelnen vorsterilisierten Plastiksack bzw. Brutsack 50 abfüllt. Nach Erreichen eines vorgewählten Sackgewichtes wird der volle Sack direkt einer sich ebenfalls unter Laminar-Flow daneben befindlichen Schweissanlage 62 übergeben, die den Sack keimdicht verschweisst. Der Sack weist als Atmungsöffnung einen speziellen Filter auf. Der Brutsack 50 wird dann in einer Etikettieranlage 63 mit allen notwendigen Informationen gekennzeichnet und verlässt die Reinraumkabine 58. Der Brutsack 50 kann nun in normaler Umgebungsluft durch einen Manipulator 64 in spezielle Transport- und Reifepaletten 65 eingelegt, in ganzen Stapeln 66 aufgeschichtet und in einen Reiferaum 67 zur notwendigen Lagerung und anschliessend in einen Kühlraum zur längeren Haltung gebracht werden. Von hier aus erfolgt deren Ablieferung an die Pilzzüchter.

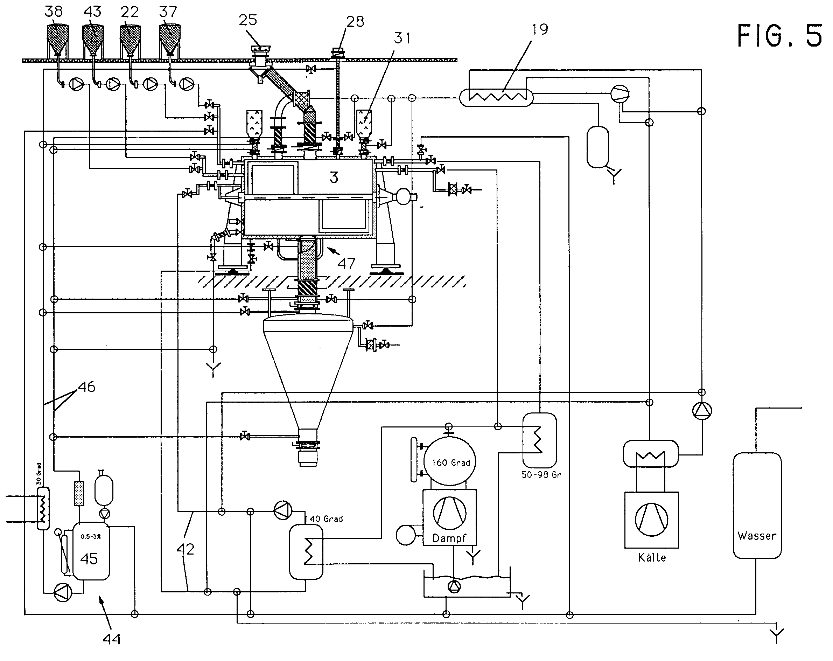

In der Folge wird nun auf die Fig. 5 Bezug genommen, welche die Führung bzw. die Kreisläufe der hauptsächlichen für das Verfahren benötigte Medien schematisch darstellt. Ein Temperatursteuerungskreislauf 42 dient dem Heizen und Kühlen der Anlageelemente. Dieser Kreislauf dient ferner einer optimalen Energieausnützung. Von der chemischen Station 44 eines Sterilisierkreislaufes 46 wird Desinfektionslösung 45 (Gemisch Desinfektionsmittel 22 mit Leitungswasser) über ein Sprühleitungssystem mit Sprühdüsen 47 an alle erforderlichen Stellen geleitet.

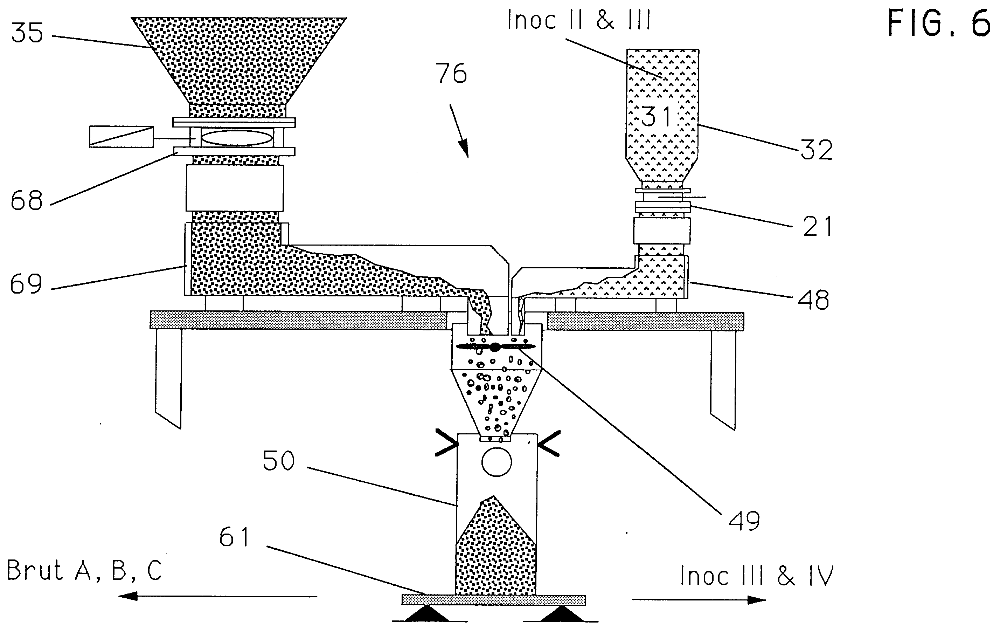

Das Anlagedetail nach Fig. 6 zeigt eine besonders vorteilhafte und erweiterte Ausgestaltung der Sackfüllstation 59. Sie dient erstens für die Herstellung von mittleren und kleinen Mengen von einzelnen Sorten von für den Handel bestimmten Brutsäcken, zweitens für die eigene Vermehrung von Inoculum 31. Einem kontinuierlichen Misch- und Dosiergerät 76 wird von dem Zwischenspeicher 35 über eine Austragsklappe 68 und eine Dosierrinne 69 aufbereitetes Brutmaterial zugeleitet. Dieser Einrichtung gegenüber ist eine ähnliche, aber in den Dimensionen wesentlich reduzierte Zufuhr- und Dosiereinrichtung plaziert. An dieser Einrichtung werden Inocbomben 32, enthaltend Inoculum 31, steril und dicht angeflanscht. Über die Austragsklappe 21 der Inocbomben und die Dosierrinne 48 wird dem Närsubstrat Inoculum dosiert hinzugefügt. Hierauf wird über den Drehtellermischer 49, welcher Nährsubstrat und Inoculum optimal vermischt, das geimpfte Nährsubstrat zur Absackung auf eine elektronische Waage 61 gegeben. Auf diese Weise kann aus reinem und teurem, im Labor gezüchteten Brutmaterial (Inoc II) durch Impfen von Nährsubstrat aus dem Zwischenbehälter 35 eine grössere Menge gleichwertigen Impfmittels (Inoc III) hergestellt bzw. abgeleitet werden. In gleicher Weise kann dieses für die Produktion von Brutmaterial erstmals abgeleitete Impfmittel (Inoc III) mit dieser Einrichtung ein zweites Mal abgeleitet und zu einem gleichwertigen Impfmittel (Inoc IV) vermehrt werden. Der Bedarf an labormässig produziertem Impfmittel (Inoc II) lässt sich somit (im Vergleich mit dem heutigen Bedarf) auf wenige Einheiten reduzieren. Weitere solche Ableitungen zur Vermehrung des Impfmittels sind nur mit Vorbehalten möglich. Inoc IV wird in der Regel für die Grossmengenproduktion und Inoc III für die Mittel- und Kleinmengenproduktion von Brutmaterial verwendet.

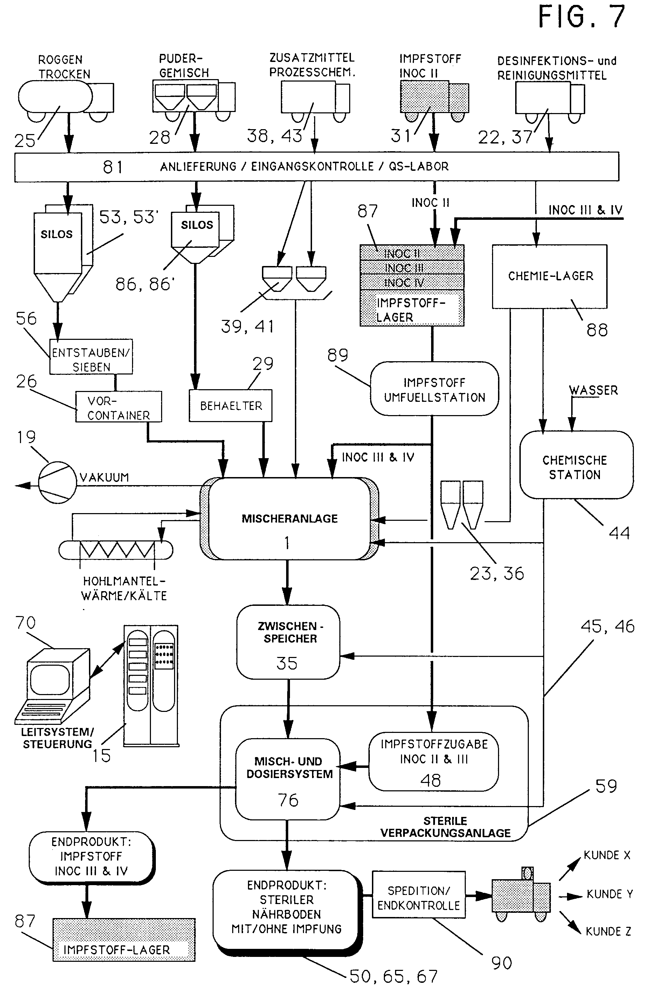

Das Blockschema nach Fig. 7 zeigt den Materialfluss in einer Anlage nach Fig. 4 von der Anlieferung bis zur Ablieferung. Wesentlich dabei ist das Erfassen von Analysewerten an allen wichtigen Schnittstellen zum Reproduzieren des Verfahrensablaufes respektiv Optimieren bei sich verändernden Parametern über eine programmierbare Steuerung 15 mit einem entsprechenden Prozessleitsystem/Speicher 70, so dass bei identisch wiederkehrenden End- und Ausgangsprodukten nach vorgewähltem Schema respektive spezifischem Rezept der ganze Prozess steuerbar ist. Besonders wichtig ist aber auch eine Eingangskontrolle 81, nicht nur der Mengen sondern aller benötigter Qualitätsparameter der Eingangsrohstoffe. Dies betrifft sowohl die Zulieferungen von Rohgetreide 25, Pudergemisch 28, Impfstoff 31 wie auch die Desinfektionsmittel 22. Das Rohmaterial wird in entsprechenden Grosslagern für Roggensilo 53 bzw. anderer Basisnährsubstrate 53' usw. Pudergemischsilo 86, 86', Impfstofflager 87 (Inoc II, III und IV) sowie Chemielager 88 auf Vorrat gehalten.

In Fig. 7 ist auch der Produktfluss für jene Impfmittel dargestellt, welche mit Inoc II, Inoc III und Inoc IV bezeichnet sind.

Da es sich beim Impfmittel Inoc II um kleine Mengen (ca. 1 Liter) einer Reinkultur von Myzel handelt, wird dieses wie vorangehend anhand von Fig. 6 beschrieben, zu Inoc III und IV vermehrt. Diese werden bis zur Reife in dem Impfstofflager 87 aufbewahrt. In einer Impfstoff-Umfüllstation 89 werden die entsprechenden Wechsel von einem Behältnis zum anderen steril vorgenommen.

Das Verfahren lässt sich nun je nach Ausbaugrad und Grösse der Anlage zeitlich und örtlich in ganz verschiedene Schritte aufteilen. Es kann für Spezialkulturen bereits sterilisiertes und gequollenes Nährsubstrat von einem anderen Produktionsort angeliefert und in dem zuvor mit chemischen Mitteln und eventuell Dampf vollständig steril gemachten Prozessraum 3, über einen Anschluss für gequollenes Nährsubstrat 75 (Fig. 1) steril eingefüllt werden. Das Nährsubstrat kann durch Beheizen des Hohlmantels auf eine Temperatur von z.B. 30 bis 40 ° C gebracht und anschliessend können, bei gleichzeitiger Bewegung der Rührarme 7 Fettsäuren 38 auf die Körner aufgetragen werden.

Anschliessend kann zur Beschichtung der Körner eine gewünschte Menge Kalkpuder von dem Puderbehälter 29 bei fortgesetzter Bewegung der Rührarme 7 in den Prozessraum eindosiert werden. Von einer zuvor aufgesetzten Inocbombe 32 wird spezielles Impfmittel in das Nährsubstrat eingemischt und das ganze Brutmaterial in den Zwischenspeicher abgegeben. Von hier aus wird es über die "Linie" für grosse und mittlere Mengen in Brutsäcke abgefüllt.

Eine zweite Variante liegt darin, die Quellung und ggf. auch die Sterilisierung räumlich dem Prozessraum 3 vorzulagern. Dies hat den Vorteil, dass der besonders zeit- und raumaufwendige Quellvorgang mit Einschluss der Sterilisierung in sehr einfachen Mischern oder Autoklaven, die nur auf Wasser- und Wäremeprozesse ausgelegt sind, durchgeführt werden kann. Bei einer solchen Trennung kann die Aufquellung und Sterilisierung in einfachen Mischern oder Autoklaven durchgeführt werden, was kostengünstiger sein kann. Hierzu können zwei oder mehrere Einheiten parallel oder in Serie geschaltet sein. Auch könnte der bakteriologisch hochsensible Anlageteil für das Beschichten und Inoculieren kleiner dimensioniert werden, weil nun die entsprechenden Phasen weniger Zeit in Anspruch nähmen, so dass der Prozessraum z.B. stündlich für eine nächste Charge bereitstellbar wäre. Die Abfüllung könnte genau gleich wie gemäss der ersten Variante erfolgen. Die örtliche Aufteilung des Prozesses ist grundsätzlich möglich, aus Qualitätsgründen (Parameter und Prozessbeherrschung) jedoch nicht empfehlenswert.

Gemäss einer dritten Variante wird das Aufquellen und Sterilisieren des Ausgangskornmaterials in dem Prozessraum 3 durchgeführt. Es kann aber auch zusätzlich das Korn mit Fettsäuren sowie mit Puder beschichtet werden. In der Folge kann zwischen zwei Möglichkeiten gewählt werden. Nur eine Teilmenge des fertig aufbereiteten Nährsubstrates wird in den Zwischenspeicher 35 abgelassen und von dort für die Herstellung von Kleinstmengen über Mikrodosierrinnen 48 mit einem Inoc II oder III geimpft. Der Rest des Nährsubstrates kann danach direkt in dem Prozessraum 3 z.B. mit Inoc III oder IV geimpft und entsprechend der "Linie" für grosse Mengen in die Brutsäcke abgefüllt werden. Die zweite Möglichkeit liegt in der Verarbeitung des Nährsubstrates in Teilmengen, welche je mit Impfmitteln unterschiedlicher Sorte geimpft werden. Dabei sind die Anlageteile zwischen jedem Wechsel mit Desinfektionsmittel steril zu reinigen.

Eine vierte Variante liegt in der vollständigen Behandlung von der Einweichung bis zur Inoculierung in dem Prozessraum 3, was in der Folge anhand von zwei ausführlichen Beispielen gemäss Fig. 3 dargestellt wird, welche die Verfahrensschritte mit 60 ° C respektiv 95 ° C Aufquelltemperatur, Hochvakuum unterstützter Abkühlung sowie einer chemischen Sterilisation beschreiben.

Beispiel 1 (TW = 60° C)

Vorbereitungsarbeiten

Roggenqualität im Labor bestimmen:

- Anfangs-Wassergehalt ($H_2O$%) des Roggens messen
- Wasseraufnahmefähigkeit messen
- Bruchanteile messen
- Roggenherkunft bestimmen
- Impfsorte definieren
- "Prozesskurve" und Mengenbilanztabelle definieren, d.h. den Prozessablauf hinsichtlich Temperatur, Druck, Rezeptur, Zugaben und Zeit festlegen: $H_2O$% = f (Tp, tv, Roggensorte), wobei Tp eine Funktion von Tw, Tm, ev.Qd und Endwert $H_2O$% (42 bis 48 %) sortenabhängig ist.

Hierbei bedeuten:

| | | |
|---|---|---|
| Tp = | Produkttemperatur | |
| Tw = | Sterlisationswassertemperatur | |
| Tm = | Hohlmantel-Temperatur (Innenwand) | |
| Td = | Temperatur Heiz-Kühlmedium im Hohlmantel | |
| Qd = | Direkt eingespiesene Dampfmenge (Temperatur-Unterstützung) | |
| tv = | Zeit, während welcher der Roggen im Heisswasserüberschuss liegt (t2-t1) | |
| Ta = | Gesamte Aufquellzeit (t3-t1). | |

Während des Prozesses werden die Soll-Ist-Mengenbilanztabellen laufend automatisch errechnet und ausgedruckt und alle wichtigen Prozessparameter gespeichert. Nach oder während der Bereitstellung der Steuerung 15 bzw. des Leitsystems 70 werden die Puderbehälter 29 mit dem bereits vorsterilisierten und vordosierten Puder 28 sowie die INOC-Bomben 32 mit dem bereits vordosierten Impfstoff auf die entsprechenden Schleusenstutzen mit Flanschanschluss der Aufquell- und Sterilisiereinheit 1 aufgesetzt.

**Phase 1**

**Schleusendesinfektion Beheizung (Dauer: 10 Min.)**

Start für den vollautomatischen Ablauf. Füllen der Schleusen 21 unter den Puderbehältern 29 und den INOC-Bomben 32 mit Desinfektionslösung 45 und Halten der Schleusentemperatur (Ts) bei max. 30° C. Füllen des Hohlmantels 2 mit Heizdruckwasser. Beheizung des Hohlmantels 2 auf Td ( = Temperatur gemessen bei Doppelmantel Eintritt), um die in der Prozesskurve vorgegebene Temperatur Tm zu erreichen. Dabei ist Tm einstellbar durch Td.

**Phase 2**

**Wasserzugabe (Dauer : 20 Min.)**

Das Tara-Gewicht der Aufquell- und Sterilisiereinheit 1 wird mittels der Wägezellen 5 erfasst. Anschliessend erfolgt das Einfüllen des Aufquellwassers über Heisswasserschieber 12 in den Prozessraum 3. Das Aufquellwasser hat eine Temperatur von TW = 60° C. Das Aufquellwasser besteht aus normalem Leitungswasser. Die Wasserzugabe wird quantitativ mittels der Wägezellen 5 erfasst.

**Phase 3**

**Zugabe der flüssigen Desinfektionsmittel und allfällig weiteren Prozesschemikalien und Roggenzugabe (Dauer: 10 Min.)**

Zugabe mittels Hahn 30 der Prozesschemikalie 43 z.B. Essigsäure, um einen pH-Wert für eine optimale Wirkung des Desinfektionsmittels einzustellen und um die Sterilisation zu unterstützen. Mengenmässig wird die Zugabe mittels der Wägezelle 5 gesteuert. Zugabe des Desinfektionsmittels 22, das mengenmässig ebenfalls mittels der Wägezellen 5 gesteuert wird. Als Desinfektionsmittel wird z.B. ein Mittel auf der Basis aktivierten Sauerstoffs, z.B. eine Wasserstoffperoxid-Lösung oder eine Peressigsäurelösung oder vorzugsweise eine Mischung beider Lösungen verwendet. Das Mittel vermischt sich mit dem Aufquellwasser zur Desinfektionslösung (0,5 bis 3 % Lösungsanteil je nach Keimbefall). Anschliessend wird der staub- und bruchfreie Roggen in den Prozessraum 3 eingespiesen. Die Roggenzugabe wird mittels den Wägezellen 5 quantitativ erfasst. Beginn Aufquellphase: $t_1$ erfassen. Halten von Tw auf dem voreingestellten Wert. Dazu kann nach Bedarf zusätzlich Reindampf mittels des Dampfschiebers 13 eingelassen werden. Erfassen der Wasserzunahme mittels der Wägezellen 5.

**Phase 4**

**Aufquellen, Sterilisieren, Ablassen (Dauer: 80 Min.)**

Aufquellen und Keimfreimachen der Getreidekörner gemäss Prozesskurve $H_2O$ = f(Tp,tv) im überschüssigen Desinfektionswasser bei Tp = mind. 60° C und tv max. 80 Min. Ein Teil des überschüssigen Wassers wird von den Körnern für das Aufquellen absorbiert. Nach Ablauf der Aufquellphase tv = $t_2$-$t_1$ hat der Roggen die erforderliche Feuchtigkeit erreicht (je nach Sorte 42 bis 48 %), was durch eine Probeentnahme überprüfbar ist.

Rasches Ablassen des überschüssigen Desinfektions- und Aufquellwassers mittels spezieller Separiereinrichtung 16 und Zurückhalten der Roggenmasse. Das Austrocknen der oben liegenden bzw. zu starkem Quellen der unten liegenden Körner, wird durch schonendes Bewegen der Rührarme 7 vermieden. Die Wasserentnahme wird ebenfalls mittels Wägezellen 5 erfasst. Weil die Ablassphase ebenfalls eine gewisse Zeitspanne ($t_3$) dauert, absorbiert der Roggen zusätzliches Wasser. Die definitive Wasseraufnahme bzw. der Endfeuchtegehalt des Roggens lässt sich mittels der Messzellen 5 genau ermitteln. In der Regel liegt die Endfeuchte bei 50 bis 52 %. Die genaue Einhaltung der Zeiten $t_1$, $t_2$, $t_3$ sowie der Mengenbilanz in der Misch- und Sterilisationseinheit 1 ist für die Qualität des Nährsubstrates von Bedeutung.

**Phase 5**

**Zusatzmittelzugabe (Dauer: 5 Min.)**

Durch Aktivierung des Hahns 40 fällt die Misch-Fettsäure-Lösung 38 in den Prozessraum 3 ein, die den Nährstoffgehalt des Nährsubstrates steigert und zugleich als Haftsubstanz für das Pudergemisch dient. Das Zusatzmittel wird vorzugsweise in flüssiger und steriler Form zugegeben. Die Zusatzmittelzugabe wird vom Wägesystem 5 erfasst. Schonende Verteilung des Zusatzmittels durch die Rührarme 7.

**Phase 6**

**Puderschleusen trocknen (Dauer: 5 Min.)**

Nach abgeschlossener Misch-Fettsäure-Zugabe werden die Puderbehälterschleusen 21 entleert. Das Desinfektionswasser wird in den Chemie-Tank zurückgepumpt und durch Hochvakuum eine Trocknung durchgeführt. Anschliessend wird eine Gewichtskorrektur am Wiegesystem vorgenommen.

**Phase 7**

**Puderzugabe 1. Teil (Dauer: 5 Min.)**

Durch Aktivierung der Puderbehälterklappen fällt eine erste Menge sterilen Puder über die trockenen Schleusen in den Prozessraum 3. Als Puder wurde z.B. ein Kalk/Gips-Gemisch verwendet.

**Phase 8**

**Puderzugabe 2. Teil (Dauer: 15 Min.)**

Die Dämpfe werden durch die Vakuumanlage laufend abgezogen, anschliessend findet eine zweite Beschickung des Nährsubstrates mit Puder aus den Puderbehältern 29 statt. Durch mehrmaliges Bewegen der Rührarme 7 ergibt sich eine gleichmässige Verteilung des Puders sowie ein gleichmässiges Coaten der Körner. Die Gewichtszunahme wird mittels der Wägezellen 5 erfasst.

**Phase 9**

**Abkühlung, Trocknung (Dauer: 35 Min.)**

Abkühlung von Tp = max. 60° C bis Tp ≦ 30° C und gleichzeitige Trocknung der Körner durch Wasserentnahme Qw mittels Anlegen von Hochvakuum (Oberflächenfeuchte und Restwasser im Prozessraum), wobei nur die im Produkt nicht gebundene Wassermenge mit dem Vakuum sicher entzogen werden kann. Die Wasserentnahme Qw wird mittels der Wägezellen 5 erfasst und der aktuelle Wassergehalt der Roggenmasse wird bis Erreichen des erforderlichen Endwertes (z.B. $H_2O$ = 42-48%) automatisch über die Wägezellen 5 überwacht. Nach Erreichen der definitiven Endfeuchte wird das Vakuum ausgeschaltet und durch Sterilfilter und Sterilluftschieber 14 langsam ausgeglichen. In der Abkühlungsphase ist darauf zu achten, dass der Hohlmantel, die Rührarme 7 und die Rührwelle 6 immer mindestens 10 bis 15° C kühler als das Produkt sind. Dies, um Ablagerungen und Verkrustungen an Metallteilen zu vermeiden. Deren Kühlung kann vorzugsweise bereits nach der Phase 4 durch eine Umschaltung des Heizkreislaufes auf das Kühlen eingeleitet werden. Zur vollständigen Abkühlung des Produktes in der Endphase kann das Kühlsystem zusätzlich mit einem Eiswasser-Kreislauf kurzgeschlossen werden, um das Temperaturgefälle Tp/Tm aufrecht zu erhalten.

**Phase 10**

**INOC-Schleusen trocknen (Dauer: 5 Min.)**

Nach abgeschlossener Trocknungsphase:

Entleeren und Trocknen der mit Desinfektionsmittel gefüllten INOC-Bombenschleusen 21 mittels Abpumpen und Anlegen von Hochvakuum.

## Phase 11

### INOC-Zugabe 1. Teil (Dauer: 5 Min.)

Durch eine Betätigung der INOC-Behälterklappen fällt eine erste Menge Impfmittel über die trockenen Schleusen in den Behandlungsraum 3.

## Phase 12

### INOC-Zugabe 2. Teil (Dauer: 15 Min.)

Abzug der Dämpfe mit Vakuumanlagen und gleichzeitig Beschicken mit dem restlichen Impfmittel aus den Bomben, wobei ein leichtes Vakuum während der Beschickung in dem Prozessraum 3 aufrecht erhalten wird. Mehrmaliges Mischen zur gleichmässigeren Verteilung des Impfmittels mittels Bewegen der Rührarme 7. Gewichtszunahme wird mittels der Wägezellen 5 erfasst.

## Phase 13

### Austragen (Dauer: 15 Min.)

Das Gesamtgewicht der Aufquell- und Sterilisiereinheit 1 wird festgestellt und anschliessend wird das Endprodukt durch die Austragsklappe 18 in den Zwischenspeicher 35 unter sterilen Bedingungen ausgetragen, was mit schonender und intermittierender Vor- und Rückwärtsrotation der Rührarme 7 unterstützt wird.

Die ausgetragene Endproduktmenge sowie die Produkterestmenge wird an die zentrale Steuerung 15 gemeldet und zusammen mit dem Rezept gespeichert.

## Phase 14

### Reinigen, Spülen (Dauer: 35 Min.)

Wenn bei der nächsten Charge ein anderes Inoculum z.B. ein Inoculum einer anderen Sorte verwendet wird, ist die Reinigung nach dem Prozess sehr wichtig. Abspülen des Mischer-Austragssystems (Nährsubstratauslass 34) mittels nicht dargestellter, automatischer Hochdrucksprühdüsen 47 und Reinigungs- und Desinfektionsflüssigkeit. Zuerst Abwasser mit Produkterückständen in die Wasserentsorgungsanlage über Sterilisier-Kreis-Rücklauf 46 abführen, dann Kreislauf umstellen und Desinfektionsflüssigkeit rekuperieren. Schliessen des Mischer-Austragssystems zur Einhaltung der sterilen Bedingungen. Abspülen, Reinigen und Ablösen von Ablagerungen des bzw. im Mischer(s), der Inoculum- und Puder-Eintragsschleusen, des Vakuumfilters und des Wasser-Feststoff-Separierfilters mittels automatischer Hochdrucksprühdüsen 47. Im Falle von Kalkablagerungen wird eine Entkalkung mit folgendem Reinigungsmittel 37 durchgeführt. Entkalkungslösung bei Tw = max. 30° C, Mischungsverhältnis:
- Ameisensäure (1 %)
- Leitungswasser (99 %)

Hohe Mischerdrehzahl einstellen, Reinigung und Entkalkung durchführen, Spülung mit Leitungswasser, Abwasser mit Produktrückständen in die Wasserentsorgungsanlage führen.

Während der Reinigungsphase des Mischers:

Schrittweise Umstellung des Kreislaufes des Hohlmantels von Kühlwasser auf Warmwasser. Meldung der Bereitschaft an die zentrale Steuerung 15. Inspektion des Mischer-Innern. Gelegentliche Öffnung der Mischer-Fronttüre und Inspektion. Zwischen einem oder einer Gruppe sich folgende Batches werden periodisch durch ein Umstellen der Kreisläufe für das Reinigungsmittel, das Warmwasser bzw. das Desinfektionsmittel der Prozessraum 3, die Zwischenspeicher 35 sowie die Schleusen, Stutzen und Anschlüsse einer vollständigen Sterilisation unterzogen. Dazu kann auch Reindampf bei 122° C eingesetzt werden. Die benötigte Zeit zur Abwicklung der Phasen 1 bis 14, d.h. die Batchdauer zur Herstellung einer sterilen, gepuderten und geimpften Roggenmasse von zirka 2'500 l beträgt zirka 295 Min., bzw. 4,9 Std. bei Tw = 60° C bzw. bei Einweichen des Kornes während 80 Min. und chemischer Sterilisation.

Beispiel 2 (TW = 95° C)

Bei diesem Beispiel sind die Vorbereitungsarbeiten und die Phasen 1, 3, 5, 6, 7, 8 und 10 bis 14 gleich wie beim ersten. Es werden folglich nur die Phasen 2, 4 und 9 beschrieben.

## Phase 2

### Wasserzugabe (Dauer: 20 Min.)

Das Tara-Gewicht der Aufquell- und Sterilisiereinheit 1 wird mittels der Wägezellen 5 erfasst. Anschliessend erfolgt das Einfüllen des Aufquellwassers in den Prozessraum 3. Das Aufquellwasser hat eine Temperatur von TW = 95° C. Das Aufquellwasser besteht aus normalem Leitungswasser. Die Wasserzugabe wird quantitativ mittels der Wägezellen 5 erfasst.

**Phase 4**

**Aufquellen, Sterilisieren, Ablassen (Dauer: 10 Min.)**

Aufquellen und Keimfreimachen der Getreidekörner gemäss Prozesskurve ($H_2O = F(Tp,tv)$) im überschüssigen Desinfektionswasser bei $Tp = max.$ 95° C und $tv = max.$ 10 Min.
Ein Teil des überschüssigen Wassers wird von den Körnern für das Aufquellen absorbiert. Nach Ablauf der Aufquellphase $tv = t_2-t_1$ hat der Roggen die erforderliche Feuchtigkeit erreicht (je nach Sorte 42 bis 48 %), was durch eine Probeentnahme überprüfbar ist.
Rasches Ablassen des überschüssigen Desinfektions- und Aufquellwassers mittels spezieller Separiereinrichtung 16 und Zurückhaltung der Roggenmasse. Das Austrocknen der oben liegenden bzw. zu starkem Quellen der unten liegenden Körner, wird durch schonendes Bewegen der Rührarme 7 vermieden. Die Wasserentnahme wird ebenfalls mittels Wägezellen 5 erfasst. Weil die Ablassphase Zeit in Anspruch nimmt ($t_3$), absorbiert der Roggen zusätzliches Wasser. Die definitive Wasseraufnahme bzw. der Endfeuchtegehalt des Roggens lässt sich mittels der Messzellen 5 genau ermitteln. In der Regel liegt die Endfeuchte bei 50 bis 52 %. Die genaue Einhaltung der Zeiten $t_1$, $t_2$, $t_3$ sowie der Mengenbilanz in der Aufquell- und Sterilisationseinheit ist für die Qualität des Nährsubstrates von Bedeutung.

**Phase 9**

**Abkühlung, Trocknung (Dauer: 55 Min.)**

Abkühlung von $Tp = max.$ 95° C bis $Tp \leq 30°$ C und gleichzeitige Trocknung der Körner durch Wasserentnahme Qw (Oberflächenfeuchte und Restwasser im Prozessraum), wobei nur die im Produkt nicht gebundene Wassermenge mit dem Vakuum sicher entzogen werden kann. Die Wasserentnahme Qw wird mittels der Wägezellen 5 erfasst und der aktuelle Wassergehalt der Roggenmasse wird bis Erreichen des erforderlichen Endwertes (z.B. $H_2O = 42$-48%) automatisch über die Wägezellen 5 überwacht.
Nach Erreichen des definitiven Endgewichtes wird das Vakuum ausgeschaltet und durch Sterilfilter und Sterilluftschieber 14 langsam ausgeglichen. In der Abkühlphase ist darauf zu achten, dass der Hohlmantel, die Rührarme 7 und die Rührwelle 6 immer mindestens 10 bis 15° C kühler als das Produkt sind. Dies, um Albagerungen und Verkrustungen an Metallteilen zu vermeiden. Deren Kühlung kann vorzugsweise bereits nach der Phase 4 durch eine Umschaltung des Heizkreislaufes auf das Kühlen eingeleitet werden. Zur vollständigen Abkühlung des Produktes in der Endphase kann das Kühlsystem zusätzlich mit einem Eiswasser-Kreislauf kurzgeschlossen werden, um das Temperaturgefälle Tp/Tm aufrecht zu erhalten.
Die benötigte Zeit zur Abwicklung der Phasen 1 bis 14, d.h. die Batchdauer, beträgt 225 Min., bzw. 3,75 Std. bei $Tw = 95°$ C bzw. bei Einweichen des Kornes während 20 Min. und chemischer Sterilisation.

**Patentansprüche**

1. Verfahren zur Aufbereitung eines sterilen körnigen Nährsubstrates mit 40 bis 60 % Wassergehalt für die Herstellung von Mikroorganismenkulturen in Reinkultur, dadurch gekennzeichnet, dass ohne Zerstörung der Fruchtkörperschale gereinigte und im Überschusswasser aufgequollene ganze Fruchtkörper wie Getreidekörner als Ausgangskornmaterial unter der Wirkung eines chemischen Mittels sterilisiert werden.

2. Verfahren nach Anspruch 1, dadurch gekennzeichnet, dass die Reinigung der Fruchtkörper die Auslese bzw. das Entfernen von Gesamtbesatz wie Steinen, nicht erwünschten Sämereien, Schmachtkörnern, Bruchkörnern, Staub im Korninneren infizierten Körnern usw. einschliesst, so dass der Anteil an Bruchkorn bzw. Gesamtbesatz im Ausgangskornmaterial weniger als 3 % bzw. 5 % vorzugsweise weniger als 1 % ausmacht.

3. Verfahren nach Anspruch 1 oder 2, dadurch gekennzeichnet, dass das Ausgangskornmaterial im Überschusswasser und bei mindestens zeitweise erhöhter Temperatur eingeweicht und unter Vermeidung eines Siedevorganges bei Atmosphärendruck, vorzugsweise bei einer Temperatur von 50 bis 99° C aufgequollen wird.

4. Verfahren nach einem der Ansprüche 1 bis 3, dadurch gekennzeichnet, dass dem Aufquellwasser als chemisches Mittel ein Desinfektionsmittel und ggf. weitere Agense zugegeben werden, so dass bis zum Erreichen eines gewünschten Wassergehaltes der Körner das Ausgangskornmaterial sterilisiert wird.

5. Verfahren nach einem der Ansprüche 1 oder 4, dadurch gekennzeichnet, dass nach Erreichen des gewünschten Wassergehaltes des Ausgangskornmaterials das Überschuss- bzw. Aufquellwasser abgelassen und die Temperatur des gequollenen Ausgangskornmaterials abgesenkt wird.

6. Verfahren nach einem der Ansprüche 1 bis 5, dadurch gekennzeichnet, dass die Einweichung im wesentlichen im Wasserüberschuss mit heissem Leitungswasser von weniger als 100° C erfolgt, und dass zum Sterilisieren des Ausgangskornmaterials diesem zeitweise heisse Luft oder heisser Dampf von über 100° C zugeführt wird, derart, dass keine thermischen Schädigungen am Ausgangskornmaterial eintreten.

7. Verfahren nach einem der Ansprüche 1 bis 6, dadurch gekennzeichnet, dass nach Abschluss der Aufquell- und/oder des Sterilisiervorganges und Entfernen des Überschusswassers die Temperatur des Basisnährstoffträgers durch Ansetzen eines Vakuums abgesenkt wird.

8. Verfahren nach Anspruch 7, dadurch gekennzeichnet, dass die Fruchtkörper zumindest leicht angetrocknet werden.

9. Verfahren nach einem der Ansprüche 1 bis 8, dadurch gekennzeichnet, dass zumindest ein Teil der Temperaturabsenkung nach dem Sterilisieren durch Abführen der Wärme mittels Kaltwasser erfolgt.

10. Verfahren nach einem der Ansprüche 1 bis 9, dadurch gekennzeichnet, dass die Fruchtkörper nach dem Sterilisieren vorzugsweise in noch warmem Zustand mit einer Haftsubstanz z.B. mit speziellen Mischfettsäuren überzogen werden, welche wenigstens 0,5 Gewichtsprozent des Nährsubstrates betragen.

11. Verfahren nach einem der Ansprüche 1 bis 10, dadurch gekennzeichnet, dass die Fruchtkörper nach dem Sterilisieren vorzugsweise in noch oberflächenfeuchtem Zustand oder nach dem Aufbringen eines Überzuges einer Haftsubstanz mit mehr als 5 Gewichtsprozenten, vorzugsweise mehr als 10 Gewichtsprozenten Pudergemisch überzogen werden.

12. Verfahren nach Anspruch 10 und 11, dadurch gekennzeichnet, dass im Überzug der einzelnen Fruchtkörper bestehend aus Haftsubstanz und/oder Puder, ein PH-Wert von näherungsweise 7 vorliegt.

13. Verfahren nach einem der Ansprüche 1 bis 12, dadurch gekennzeichnet, dass die Aufbereitung des Nährsubstrates im Batchverfahren erfolgt, wobei die Prozessphasen aufgrund eines vorgegebenen Rezeptes durch Zeitvorgaben und durch wiederholtes Erfassen des Batchgewichtes gesteuert werden.

14. Verfahren nach Anspruch 13, dadurch gekennzeichnet, dass es in einer Aufquell- und Sterilisiereinheit erfolgt, die auf Wägezellen abgestützt ist und im Prozessraum schonend gerührt wird, wobei in der Einweichphase langsam und während dem Beschichten schneller gerührt wird.

15. Verfahren nach einem der Ansprüche 1 bis 14, dadurch gekennzeichnet, dass die Inoculierung des Nährsubstrates wahlweise in den Prozessraum der Aufquell- und Sterilisiereinheit durch Einmischen von 0,5 bis 8 % eines Inoculums in die ganze Nährsubstratmenge oder in einem getrennten Mischer erfolgt.

16. Verfahren nach Anspruch 15, dadurch gekennzeichnet, dass Mengen des Nährsubstrates als Teilmengen aus dem Inhalt der Aufquell- und Sterilisiereinheit für die Inoculierung in dem Mischer wählbar ist.

FIG. 1

EP 0 588 145 A2

FIG. 2

$H_2O$ (%)

$T_M, T_W$ (°C)

FIG. 3

$T_M$ (Hohlmantel) bei $T_W = 95°C$

$T_M$ bei $T_W = 60°C$

Aufquellen, Sterilisieren bei 95°C sehr schnell, dafür längere Abkühlungszeit.

Aufquellen, Sterilisieren bei 60°C langsam, dafür Abkühlungszeit.

120

110

100

95°C

90

80

70

$T_W = 95°C$

60

60°C

$T_W = 60°C$

$t_1$

$t_V$

$t_2$

$t_A$

$t_3$

52%

50%

50

48%

40

30

$H_2O$-Gehalt bei 95°$T_W$

$H_2O$-Gehalt bei 60°$T_W$

20

~13%

10

Start Automatik

$T_M = \sim 4-5°C$

$t(h)$

~295 Min ≙ 4.9 Std (bei $T_W = 60°C$)

Phase  0  1  2  3  4.1  4.2  4.3  5 6 7 8  9.1  9.2 10 11  12  13  14

Gültig für Kurve $T_W$ 60°C

EP 0 588 145 A2

FIG. 4

38  43  22  37        25        28        31                    19        FIG. 5

46

50 Grad

0.5-3R
45

42

44

160 Grad

140 Grad

Dampf

50-98 Gr

Kälte

Wasser

EP 0 588 145 A2

FIG. 6

35

Inoc II & III

76

31

32

68

21

69

48

50

49

61

Brut A, B, C

Inoc III & IV

EP 0 588 145 A2

# FIG. 7

ROGGEN TROCKEN — 25
PUDER-GEMISCH — 28
ZUSATZMITTEL PROZESSCHEM. — 38, 43
IMPFSTOFF INOC II — 31
DESINFEKTIONS- und REINIGUNGSMITTEL — 22, 37

81 ANLIEFERUNG / EINGANGSKONTROLLE / QS-LABOR

INOC II
87
INOC III & IV

SILOS 53, 53'
SILOS 86, 86'
INOC II / INOC III / INOC IV / IMPFSTOFF-LAGER
CHEMIE-LAGER 88

56
39, 41
89
ENTSTAUBEN/ SIEBEN
26
VOR-CONTAINER
BEHAELTER 29
IMPFSTOFF UMFUELLSTATION
WASSER

19 VAKUUM
INOC III & IV
CHEMISCHE STATION 44

MISCHERANLAGE 1
HOHLMANTEL-WÄRME/KÄLTE
23, 36

70
LEITSYSTEM/ STEUERUNG 15
ZWISCHEN-SPEICHER 35
45, 46

MISCH- UND DOSIERSYSTEM 76
IMPFSTOFFZUGABE INOC II & III 48
STERILE VERPACKUNGSANLAGE 59

ENDPRODUKT: IMPFSTOFF INOC III & IV
ENDPRODUKT: STERILER NÄHRBODEN MIT/OHNE IMPFUNG 50, 65, 67
SPEDITION/ ENDKONTROLLE
KUNDE X / KUNDE Y / KUNDE Z
90

87
IMPFSTOFF-LAGER

18